Europäisches Patentamt

European Patent Office

Office européen des brevets

(19)

(11) Publication number: **0 067 702**
**B1**

# EUROPEAN PATENT SPECIFICATION

(12)

(45) Date of publication of patent specification: **05.02.86**

(21) Application number: **82303077.0**

(22) Date of filing: **14.06.82**

(51) Int. Cl.⁴: **A 61 B 17/36,** H 02 H 5/10

(54) Laser scalpel including a safety device.

(30) Priority: **16.06.81 JP 91516/81**

(43) Date of publication of application:
**22.12.82 Bulletin 82/51**

(45) Publication of the grant of the patent:
**05.02.86 Bulletin 86/06**

(84) Designated Contracting States:
**DE FR GB SE**

(56) References cited:
**US-A-3 642 007**
**US-A-3 838 242**

(73) Proprietor: **SUMITOMO ELECTRIC INDUSTRIES LIMITED**
**No. 15, Kitahama 5-chome Higashi-ku**
**Osaka-shi Osaka 541 (JP)**

(72) Inventor: **Yoshida, Kenichi**
**Osaka Works of Sumitomo Electr. Ind. Ltd.**
**1-3 Shimaya 1-Chome Konohana-ku Osaka (JP)**
Inventor: **Hiramoto, Junichi**
**Osaka Works of Sumitomo Electr. Ind. Ltd.**
**1-3 Shimaya 1-Chome Konohana-ku Osaka (JP)**
Inventor: **Takenaka, Shinya**
**Osaka Works of Sumitomo Electr. Ind. Ltd.**
**1-3 Shimaya 1-Chome Konohana-ku Osaka (JP)**

(74) Representative: **Rackham, Stephen Neil et al**
**GILL JENNINGS & EVERY 53-64 Chancery Lane**
**London WC2A 1HN (GB)**

## Description

This invention concerns a laser scalpel including a safety device. This makes the laser scalpel very safe for both patients and operators.

A laser scalpel works by taking advantage of high energy power of lasers such as carbon dioxide gas lasers and others; therefore, thers is always need for careful handling of it, particularly when it is used in the field of medical care.

Generally, any medical instrument is designed to be handled so as not to endanger patients, but this does not necessarily mean that a malfunction or breakdown resulting in an accident never occurs, so strict safety measures are required. When a laser instrument is used, special attention must be paid to operations, with it, because high tension circuits are built into it and, further, a laser beam is invisible in most cases.

Most electronic instruments have grounding terminals to ensure safe handling and operation. As electronic instruments are increasingly put to use in various industrial fields, some products have become available on the market which require positive grounding. For example, there is an instrument for medical engineering use, which holds high tension circuits inside. Therefore, prior to its use, in addition to connecting the casing of the electronic instrument to the grounding terminals arranged on the floor and some other places where grounding wires, an electric cord, containing a grounding wire other than wires for power supply, is plugged in a socket so as to ensure the contact of the electronic instrument with another grounding wire in the power source side.

This seems satisfactory; however, if the grounding wires are disconnected, by accident or otherwise, or if the socket should not be effectively earthed, there is risk of an accident. From this viewpoint, it is true that, until today, adequate measures to cope with problems of this kind have not been taken, and a laser instrument for medical use cannot be considered an exception.

There are several conventional methods of measuring current leakage in an electronic instrument. For example, one method is to convert the difference in electric current flowing from the power source to the electric instrument and from the electric instrument to the power source into intensity of magnetic flux to detect the leakage current in the form of an induced electric current. However, as the loss in the conversion is too great to detect the leakage current accurately, the disadvantage of this method of measurement is that it can only be applied to the case when the leakage current is great—such as a current exceeding 10 mA. Consequently, in the case of an electronic instrument in which it is necessary to detect a very small difference in leakage current ranging from some tens of microamperes to some hundreds of microamperes, there is no proper way of detection.

Therefore, an object of this invention is to provide a laser scalpel which is very safe in use for both patients and operators.

According to this invention a laser scalpel apparatus including a laser, a laser power source, a casing encasing the laser scalpel apparatus, and an optical duct for the laser beam also includes a safety device which comprises a group of detectors including a grounding disconnection detector which monitors the electrical connection between the laser scalpel power source and earth and a current leakage detector which monitors current leakage between the optical duct and the casing, a laser power source breaker, an alarm system and a signal processing system which receives signals emitted by the two detectors and outputs signals to operate the alarm system and the laser power source circuit breaker to isolate the laser from its power source when the laser power source is disconnected from earth or when current leakage occurs between the optical duct and the casing.

A particular example of laser scalpel in accordance with this invention will now be described with reference to the accompanying drawings; in which:—

Figure 1 is a block diagram of the laser scalpel; and,

Figure 2 is a circuit diagram of the grounding disconnection detector.

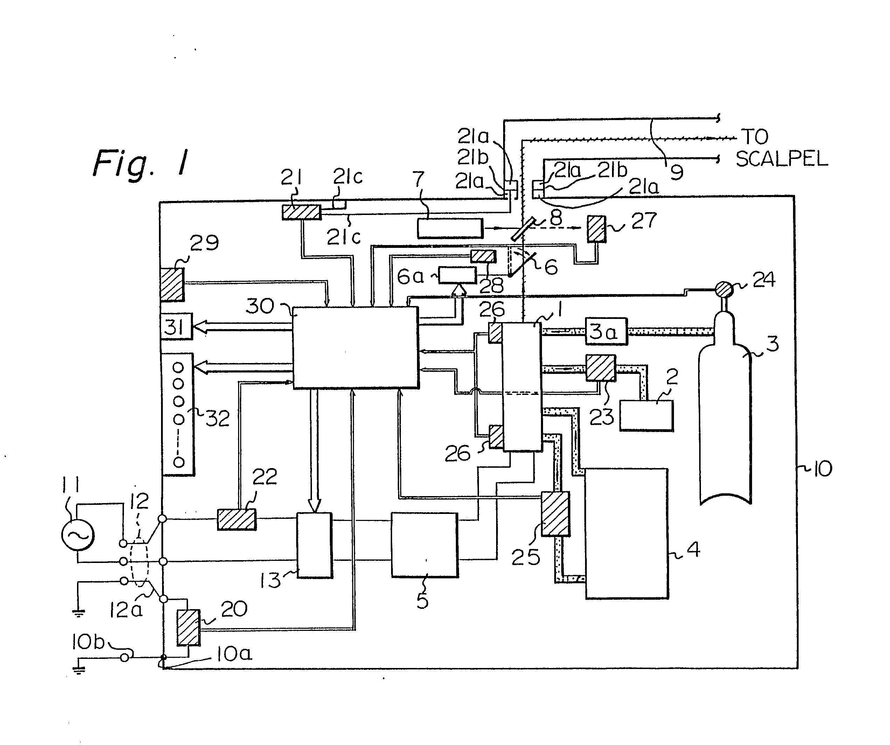

Fig. 1 shows one of the preferred embodiments of this invention. For caution's sake, it had better be stated here that the scalpel part is not shown in Fig. 1 although this drawing illustrates the example of a laser scalpel which utilizes a $CO_2$ laser, Ar laser, or other similar kind of lasers. The mechanism of generating a laser beam will be outlined up to the scalpel part.

A gas laser 1, while being cooled with a coolant sent from a coolant-circulating apparatus 4, is impressed with high voltage from high tension power source 5 of laser use when a chamber of the gas laser is refilled up to a desired pressure with $CO_2$ or Ar-mixed gas sent from a gas cylinder 3 after the chamber has been evacuated by means of a vacuum pump 2. An invisible laser beam emitted from the gas laser 1, after having passed through a shutter 6, is combined with a collimating visible beam, like He-Ne laser beam emitted from a laser 7, by means of a half-silvered mirror 8, and then led to the scalpel part by way of an optical duct 9 for passing the laser beam, wherein numeral 3a shows a gas-regulating mechanism, numeral 6a a shutter-perating mechanism, numeral 10 a metal casing, numeral 11 a commercial power source, numeral 12 an electric cord containing a grounding wire, and numeral 13 a laser power source breaker.

In Fig. 1, elements hatched and numbered with from 20 to 29 are different kinds of detectors put to use to find abnormalities. A signal-processing system 30 is designed to receive every signal emitted from the above detectors, and to activate an alert buzzer 31 and an alert lamp panel 32, to alert operators anytime to keep safe operation, the laser power source breaker 13, and the shutter-operating mechanism 6a. Numeral 20 shows a detector for seeing whether the casing 10 is grounded; numeral 21 shows a detector for seeing whether there is a leakage of current en route from the casing 10 to the scalpel which is to be brought into contact with the body of patients. These detectors will be described later in more detail. Numeral 22 shows a detector for seeing whether a laser discharge current is too great, which can be built up of a lead switch and a simple comparison circuit. Numeral 23 shows a detector for seeing whether there is any abnormality in the pressure of $CO_2$ and Ar-mixed gas. Numeral 24 shows a detector for seeing whether the residual pressure of a gas cylinder 3 is too low. These detectors work by means of a pressure-sensing switch or a unit assembled from a pressure oscillator and a comparison circuit. Numeral 25 shows a detector, which can be made up of a flow meter and a pressure-sensing switch, for seeing whether the amount of coolant flowing is too little. Numeral 26 shows a detector for seeing whether the electrodes of the gas laser are overheating. This can be made up of a temperature-sensing switch, or assembled from a thermistor and a comparison circuit. Numeral 27 shows a detector for seeing whether there is any abnormality in a laser 7 emitting a visible beam for collimation; this detector can be assembled from photo detectors, such as a photo-transistor, a photodiode, and the like which serve to receive the collimation beam having passed through a half-silvered mirror 8, and a comparison circuit. Numeral 28 shows a detector for seeing whether there is any abnormality in the movement of the shutter 6, which can be constructed from a unit that serves to check a voltage being impressed on a micro-switch, and a solenoid built in the shutter-operating mechanism 6a by means of a comparison circuit. Numeral 29 shows a detector for seeing whether a window of the casing is properly locked, which can be made up of a micro-switch.

Alert lamps (10) alert lamps are needed in this example), each of which is correspondent to each of the above detectors numbered from 20 to 29, are arranged on the alert lamp panel 32, and a name plate is attached to the respective alert lamps to show the corresponding detector. The signal-processing system 30 is built up of a micro-computer, for example. The system carries out the following judgements and controls in order to keep safe operation while receiving a variety of signals from the different detectors from 20 to 29.

1) The signal-processing system causes the buzzer 31 to give an alarm whichever detector picks up abnormality in its own region.

2) The signal-processing system causes the alert lamps to flicker to tell the position where abnormality is happening.

3) The signal-processing system causes the laser power source breaker 13 to operate so as to stop the emission of the laser beam if there is grounding wire disconnection, current leakage, excessive laser discharge, pressure drop in the flowing coolant gas mixture, overheating of the laser electrodes, extinction of the collimation beam, or malfunctioning of the shutter.

In this example, the system simultaneously gives the shutter-operating mechanism in order to stop the laser beam and prevent the beam from getting to the scalpel if the laser should not stop emitting the beam. Additionally, in the case of minute malfunctions like the window of the casing being left open, or pressure of the gas cylinder dropping, the system causes only the buzzer to give an alarm; thus, the whole system is able to continue operation without using the laser power source breaker 13 or the shutter-operating mechanism 6a, because such malfunctions can be corrected quickly with ease.

With all of the above mechanisms, the safety surveillance system is made complete with respect to both the fundamental laser operation and to the functions required for safe operation as a medical instrument; in consequence, this system can provide a highly reliable laser scalpel.

In Fig. 1, the hatched arrow (—/—/—→) shows the laser beam from a $CO_2$ or Ar gas laser; the solid arrow (———→) shows a collimation laser beam from a He—Ne laser; and the dotted arrow (—---→) shows the same collimation laser beam transmitted by the half-silvered mirror.

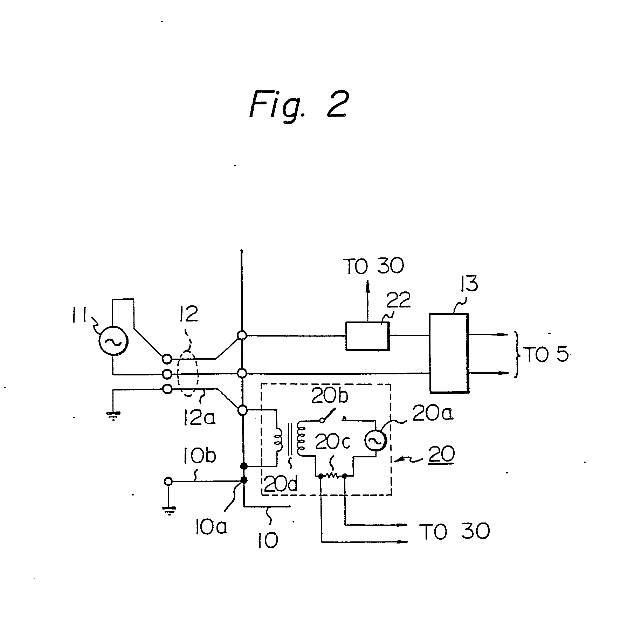

One of the examples of the grounding disconnection detector 20 is shown in Fig. 2, in which numeral 20a shows an alternating current power source for test use, numeral 20b a switch, numeral 20c a resistance, 20d a transformer for test use. The primary side of the transformer 20d is connected to the AC power source 20a; the resistance 20c and the switch 20b are also connected to the primary side to make up a closed circuit; besides one end of the secondary side of the transformer is connected to the grounding wire 12a in the electric cord 12, while the other end of the secondary side is connected to a grounding terminal 10a on the casing 10. Because the casing 10 is generally connected to a grounding terminal provided in the wall of a room by means of a grounding wire 10b, the secondary side of the transformer 20d is short-circuited as long as the ground is complete; therefore, a large current flows in the primary side and the voltage between the two terminals of the resistance 20c is also great. On the contrary, however, when the grounding wire 10b or 12 is broken, disconnected, or left unconnected, the current in the primary side becomes very small, and the voltage between the terminals of the resistance 20c is substantially lowered. Therefore, by watching the voltage between the terminals of the resistance 20c and the current flowing therein, ground disconnection can be detected.

On the other hand, in this example of the current leakage detector 21, an insulator 21a is put between the optical duct 9 and the casing 10; moreover, the casing 10 is connected to electrodes 21b embedded in the insulator 21a by

the use of a wire 21c to detect a current flowing in this wire.

The optical duct 9 for the laser beam is usually in a cylindrical form and is made of electroconductive carbon fiber, so there is a likelihood of current leakage. And if a patient moves suddenly upon receiving an electric shock when touched by the laser scalpel, a serious accident might happen. However, with this system, if there should be a current leakage through the insulator 21a to an optical duct 9, the current flows mainly in electroconductive wire 21c, so that even a very small current leakage can be detected. In this way, patients can be fully free from an electric shock.

## Claims

1. A laser scalpel apparatus including a laser (1), a laser power source (5), a casing (10), enclosing the laser scalpel apparatus, and an optical duct (9) for the laser beam, characterised in that it includes a safety device which comprises a group of detector including a grounding disconnection detector (20) which monitors the electrical connection between the laser scalpel power source (5) and earth and a current leakage detector (21) which monitors current leakage between the optical duct (9) and the casing (10), a laser power source circuit breaker (13), an alarm system (31, 32), and a signal processing system (30) which receives signals emitted by the two detectors (20 and 21) and outputs signals to operate the alarm system (31, 32) and the laser power source circuit breaker (13) to isolate the laser (1) from its power source (5) when the laser power source (5) is disconnected from earth or when current leakage occurs between the optical duct (9) and the casing (10).

2. A laser scalpel according to claim 1, which also includes a laser beam shutter (6), and in which the alarm system includes a buzzer (31) and alarm lamps (32) corresponding to each of the group of detectors, the signal processing system (30) sending output signals for operating the buzzer (31) and, at the same time, flashing the alarm lamp (32) corresponding to the fault detected, the signal processing system (30) also sending a signal to close the laser beam shutter (6).

3. A laser scalpel according to claim 2, in which the group of detectors also includes a detector (28) for detecting operational failure of the laser beam shutter (6).

4. A laser scalpel according to claim 1 or 2, in which the group of detectors also includes an overcurrent detector (22) for monitoring the laser discharge current.

5. A laser scalpel according to any one of the preceding claims, in which the group of detectors also includes a detector (23, 24) for detecting an abnormal gas pressure in the laser (1).

6. A laser scalpel according to any one of the preceding claims, in which the group of detectors also includes a detector (25) for monitoring a decrease in the amount of a flowing coolant.

7. A laser scalpel according to any one of the preceding claims, in which the group of detectors also includes a detector (26) for detecting overheating in electrodes of the laser (1).

8. A laser scalpel according to any one of the preceding claims, in which the grounding disconnection detector (20) comprises a transformer (20d) with its primary winding connection in a closed loop with an alternating current power source and a resistance (20c) and its secondary winding having one end connected to an earthing wire (12a) of an electric power cable (12) and the other end connected to the casing (10), a grounding disconnection being detected by a drop in the voltage occurring across the resistance (20c), and in which the current leakage detector (21) includes an insulator (21a) in between the casing (10) and the optical duct (9) for the laser beam with a conductor (21b) embedded in the insulator (21a), and means (21) to detect current flow between the conductor (21b) and the casing (10).

## Patentansprüche

1. Eine Laserskalpellvorrichtung mit einem Laser (1), einer Laserversorgungsspannungsquelle (5), einem Gehäuse (10), welches die Laserskalpelleinrichtung enthält, und einem optischen Leiter (9) für den Laserstrahl, dadurch gekennzeichnet, daß sie eine Sicherheitseinrichtung enthält, welche eine Gruppe von Detektoren, enthaltend einen Erdanschlußtrennungsdetektor (20), der die elektrische Verbindung zwischen der Laserskalpellversorgungsspannungsquelles (5) und Masse überwacht und einen Leckstromdetektor (21), der einen Leckstrom zwischen dem optischen Leiter (9) und dem Gehäuse (10) überwacht, einen Schalter (13) für die Laserversorgungsspannungsquelle, ein Alarmsystem (31, 32) und eine Signalverarbeitungssystem (30), welches die von den beiden Detektoren (20 und 21) ausgesendeten Signale empfängt und Signale abgibt zur Betätigung des Alarmsystems (31, 32) und des Schalters (13) für die Laserversorgungsspannungsquelle zum Isolieren des Lasers (1) von seiner Versorungsspannungsquelle (5), wenn die Laserversorgungsspannungsquelle (5) von Masse getrennt ist oder wenn ein Leckstrom zwischen dem optischen Leiter (9) und dem Gehäuse (10) auftritt aufweist.

2. Ein Laserskalpell nach Anspruch 1, welches ferner einen Laserstrahlverschluß (6) enthält und in welchem das Alarmsystem einen Summer (31) und Alarmlampen (32), die den Gruppen der Detektoren entsprechen, enthält, wobei das Signalverarbeitungssystem (30) Ausgangssignale aussendet zur Betätigung des Summers (31) und gleichzeitig zum Einschalten der Alarmlampe (32), welche dem festgestellten Fehler entspricht, und das Signalverarbeitungssystem (30) ebenfalls ein Signal zum Schließen des Laserstrahlverschlusses (6) sendet.

3. Ein Laserskalpell nach Anspruch 2, in welchem die Detektorengruppe ferner einen Detektor (28) enthält zum Feststellen eines Betriebsfehlers des Laserstrahlverschlusses (6).

4. Ein Laserskalpell nach Anspruch 1 oder 2, in welchem die Detektorengruppe ferner einen Überstromdetektor (22) aufweist zur Überwachung des Laserentladungsstroms.

5. Ein Laserskalpell nach einem der vorstehenden Ansprüche, bei welchem die Detektorengruppe ferner einen Detektor (23, 24) enthält, zum Feststellen eines anormalen Gasdruckes im Laser (1).

6. Ein Laserskalpell nach einem der vorstehenden Ansprüche, bei welchem die Detektorengruppe ferner einen Detektor (25) aufweist zur Überwachung einer Abnahme der Menge an fließendem Kühlmittel.

7. Ein Laserskalpell nach einem der vorstehendem Ansprüche, bei welchem die Detektorengruppe ferner einen Detektor (26) aufweist, zum Feststellen von Überhitzung bei Elektroden des Lasers (1).

8. Ein Laserskalpell nach einem der vorstehenden Ansprüche, bei welchem der Masseanschlußtrennungsdetektor (20) einen Transformator (20d) aufweist, dessen Primärwicklungsanschluß in einer geschlossenen Schleife mit einer Wechselspannungsquelle und einem Widerstand (20c) liegt und dessen Sekundärwicklung mit einem Ende an einem Massedraht (12a) eines elektrischen Leistungskabel (12) und mit dem anderen Ende an das Gehäuse (10) angeschlossen ist, wobei eine Masseabtrennung erfaßt wird durch einen Spannungsabfall am Widerstand (20c) und in welchem der Leckstromdetektor (21) einen Isolator (21a) zwischen dem Gehäuse (10) und dem optischen Leiter (9) für den Laserstrahl mit einem im Isolator (21a) eingebetteten Leiter (21b) sowie Mittel (21) zur Erfassung eines Stromflusses zwischen dem Leiter (21b) und dem Gehäuse (10) aufweist.

**Revendications**

1. Appareil de scalpel à laser, comprenant un laser (1), une source (5) d'alimentation dudit laser, un boîtier (10) renfermant ledit appareil de scalpel à laser, et un conduit optique (9) pour le faisceau laser, caractérisé en ce qu'il comporte un dispositif de sécurité comprenant un ensemble de détecteurs dont un détecteur (20) de déconnexion de terre qui surveille la connexion électrique entre la source (5) d'alimentation du scalpel à laser et la terre et un détecteur (21) de courant de fuite vérifiant l'absence de courant de fuite entre le conduit optique (9) et le boîtier (10), un disjoncteur (13) du circuit de la source d'alimentation du laser, un système d'alarme (31, 32), et un système (30) de traitement de signaux qui reçoit les signaux de sortie des deux détecteurs (20 et 21) et délivre des signaux de déclenchement du système d'alarme (31, 32) et du disjoncteur (13) du circuit de la source d'alimentation du laser afine de couper le laser (1) de sa source d'alimentation (5) quand celle-ci est déconnectée d'avec la terre ou quand une fuite de courant se produit entre le conduit optique (9) et le boîtier (10).

2. Scalpel à laser suivant la revendication 1, comprenant en outre un organe d'obturation (6) pour l'arrêt du faisceau laser, et dans lequel le système d'alarme comprend un ronfleur (31) et des lampes d'alarme (32) correspondant à chacun des détecteurs précités, le système (30) de traitement de signaux délivrant des signaux pour le déclenchement du ronfleur (31) et, en même temps, pour le scintillement de la lampe d'alarme (32) correspondant à la panne détectée, le système (30) de traitement de signaux délivrant en outre un signal de fermeture de l'organe obturateur (6) d'arrêt du faisceau laser.

3. Scalpel à laser suivant la revendication 2, dans lequel ledit ensemble de détecteurs comprend en outre un détecteur (28) de défaillance dans le fonctionnement de l'organe obturateur (6) d'arrêt du faisceau laser.

4. Scalpel à laser suivant la revendication 1 ou 2, dans lequel ledit ensemble de détecteurs comprend en outre un détecteur de surintensité (22) surveillant le courant de décharge du laser.

5. Scalpel à laser suivant l'une quelconque des revendications précédentes, dans lequel ledit ensemble de détecteurs comprend en outre un détecteur (23, 24) pour la détection d'une pression de gaz anormale dans le laser (1).

6. Scalpel à laser suivant l'une quelconque des revendications précédentes, dans lequel ledit ensemble de détecteurs comprend en outre un détecteur (25) surveillant l'absence d'une diminution de la quantité de fluide de refroidissement en circulation.

7. Scalpel à laser suivant l'une quelconque des revendications précédentes, dans lequel ledit ensemble de détecteurs comprend en outre un détecteur (26) de surchauffe des électrodes du laser (1).

8. Scalpel à laser suivant l'une quelconque des revendications précédentes, dans lequel le détecteur (20) de déconnexion de terre comprend un transformateur (20d) dont la borne d'enroulement primaire est insérée dans un circuit fermé comprenant une source de courant alternatif et une résistance (20c), et dont une extrémité de l'enroulement secondaire est reliée à un conducteur de terre (12a) d'un câble électrique (12) tandis que l'autre extrémité dudit enroulement secondaire est connectée au boîtier (10), une déconnexion de terre étant détectée par une diminution de tension se produisant aux bornes de la résistance (20c), et dans lequel le détecteur (21) de courant de fuite comprend un isolateur (21a) entre le boîtier (10) et le conduit optique (9) de faisceau laser, un conducteur (21b) étant incorporé dans l'isolateur (21a), et des moyens (21) de détection d'un courant s'écoulant entre le conducteur (21b) et le boîtier (10).

Fig. 1

TO SCALPEL

# Fig. 2